# EUROPEAN PATENT SPECIFICATION

(11) **EP 3 075 314 B1**
(45) Date of publication and mention of the grant of the patent: **12.06.2024**
(21) Application number: 16161015.9
(22) Date of filing: 17.03.2016
(51) Int. Cl.: A61B 5/12, A61B 5/00

(54) **REMOTE USER INTERFACE WITH EAR SELECTION SWITCH**
FERNBENUTZERSCHNITTSTELLE MIT OHRWAHLSCHALTER
INTERFACE UTILISATEUR À DISTANCE AVEC COMMUTATEUR DE SÉLECTION DE L'OREILLE

(30) Priority: 19.03.2015 DK 201570157
(43) Date of publication of application: 05.10.2016
(73) Proprietor: Natus Medical Incorporated, Pleasanton, CA 94566 (US)
(72) Inventor: Lantz, Johannes, 217 64 Malmö (SE)
(74) Representative: Guardian IP Consulting I/S

(56) References cited:
- US-A- 4 060 701
- US-A- 5 699 809
- US-A1- 2007 112 279
- US-A1- 2014 309 549
- US-B1- 6 350 243
- MaicoDiagnostics: "MAICO EroScan Pro", YouTube, 19 April 2013 (2013-04-19), page 1, XP054976687, Retrieved from the Internet: URL:https://www.youtube.com/watch?v=tRFdAd nzpDI [retrieved on 2016-07-20]
- Anonymous: "Operating Instructions ERO SCAN Pro", MAICO , 2013, pages FP-iii,1-68, XP002760027, Retrieved from the Internet: URL:http://meddevicedepot.com/PDFs/eroscan _pro_operatingmanual.pdf [retrieved on 2016-07-18]
- Anonymous: "Operating Instructions MAICO MI 44", MAICO , November 2014 (2014-11), page 68PP, XP002760028, Retrieved from the Internet: URL:http://www.grilligroup.com/wordpress/w p-content/uploads/2014/11/MI-44_Manuale-ut ente.pdf [retrieved on 2016-07-18]

## Description

### FIELD OF TECHNOLOGY

A new remote user interface for an audiologic test apparatus, such as a middle ear analyser, is provided.

### BACKGROUND

In order to perform audiologic tests, such as tympanometry, ipsi and contra acoustic reflex measurements, otoacoustic emission measurements, pure tone audiometry, etc., an ear probe is provided for positioning in the ear canal, typically, for provision of a selected air pressure in the ear canal and/or a selected pure tone or other sound and/or for measurement of the sound pressure level in the ear canal. The audiologic tests are controlled by an audiologic test apparatus connected with the ear probe and the measurement results are recorded by the audiologic test apparatus. A hand-held ear probe may be used for screening purposes, while an ear probe that is retained more securely in or at the ear canal of a human is typically preferred for clinical tests. "Operating Instructions ERO SCAN Pro", MAICO, 2013, pages FP-iii,1-68 discloses a remote user interface for an audiologic test apparatus.

### SUMMARY

When clinical or screening tests are performed, there is a need for recording which of the left ear and right ear that is subjected to the test in question.

For this purpose, a switch is conventionally provided at the audiologic test apparatus for selection of which of the left ear and the right ear that is subjected to a test. For example, a reflex test may be performed, wherein the left ear is selected to be tested and the selection may be displayed on a screen of the audiologic test apparatus as "Reflex: Left".

However, it is cumbersome and time consuming for the operator of an audiologic test apparatus with an ear probe to have to move away from the patient in order to select which ear that is going to be tested and in order to select the other ear when testing of the first ear has been performed. Time is wasted and communication with the patient may be interrupted and the calm atmosphere that is desired during audiologic testing may be disturbed.

In order to eliminate the need for the operator of the audiologic apparatus and the ear probe, to move proximate the audiologic test apparatus during testing, a new remote user interface is provided that is connected with the audiologic test apparatus and that has a remote user interface housing accommodating a switch for selection of the right ear or the left ear of the patient for testing with the ear probe. During use, the remote user interface housing is positioned proximate the patient.

The remote user interface housing is preferably configured for mechanical connection with the ear probe of the audiologic test apparatus in such a way that the remote user interface will be located proximate the patient during the audiologic testing of the patient. With the remote user interface located proximate the patient, e.g. worn by the patient, the operator of the ear probe may perform selection of the ear to be subjected to the test, or being subjected to the test, without being forced to move proximate to the audiologic test apparatus connected with the ear probe for the purpose of ear selection.

For example, an operator of an audiologic test apparatus with an ear probe performing audiologic testing of a patient's ears may recognize, after having positioned the ear probe appropriately in the desired ear of the patient that he or she has not selected the correct ear for testing as required to be able to record the test results together with identification of the selected ear, i.e. correct information on whether the test results relate to the right ear or the left ear. Conventionally, the operator in this situation had to move away from the patient to the audiologic test apparatus to perform the selection of the right ear or left ear and revert to the patient and possibly check or even re-establish the desired positioning of the ear probe. However, the operator desires to maintain focus on the patient and the patient's reactions and wants to minimize disturbing activities, such as moving away from the patient. This is avoided with the new remote user interface.

Preferably, the switch for selection of the right ear or the left ear for testing with the ear probe does not provide other functions in response to its actuation, for example the switch is not used for simultaneously control of the start and stop of the audiologic test.

Preferably, the switch is a momentary switch also called a mechanically non-latching switch, i.e. the switch reverts to the same mechanical state or position upon activation as for example a spring biased push button switch. The mechanically non-latching switch may be a touch sensitive switch that is actuated by touching its surface with a finger and that provides a switch signal when actuated, e.g. by making contact between two poles of the switch or by breaking contact between two poles of the switch, or in any other suitable way well-known in the art of touch sensitive switches.

The touch sensitive switch may be positioned so that a surface of the touch sensitive switch is flush with a surface of the remote user interface housing.

The touch sensitive switch may have a surface that forms a part of a surface of the remote interface housing.

The mechanically non-latching switch may be a push button switch, e.g. biased with a spring, e.g. with appropriate tactile feedback, when the switch is actuated, i.e. depressed or released, and that provides a switch signal when actuated, e.g. by making contact between two poles of the switch or by breaking contact between two poles of the switch, or in any other suitable way well-known in the art of push buttons.

A mechanically latching switch has a moving part called the actuator with two or more mechanical states or positions and changes mechanical state or position upon actuation. The switch remains in its changed state or position until the next actuation is performed. For example, the well-known toggle switch or flip switch, e.g. a wall switch, has two mechanical states or positions, and a user actuates the switch by changing its position for example to turn on a lamp in one position and turn the lamp off in another position.

The mechanically non-latching switch may be configured for alternating selection of the right ear or the left ear for testing with the audiologic test apparatus by repeated actuation of the switch.

The remote user interface housing may accommodate two switches of the same or different ones of the above-mentioned types of mechanically non-latching switches, each of which is provided for selection of the right ear or the left ear, respectively.

The remote user interface housing may accommodate one or two switches of the above-mentioned types of mechanically non-latching switches configured for controlling start and stop of testing with the audiologic apparatus as is well-known in the art.

The remote user interface housing may accommodate an indicator of the one of the right ear and left ear that is currently selected for testing with the ear probe.

The indicator may provide visual indication of the selected ear, e.g. the indicator may comprise a light source, such as a lamp, a LED, etc., that turns on when one of the right ear and left ear is selected for testing and that turns off when the other one of the right ear and left ear is selected for testing.

The indicator may comprise a light source, such as a lamp, a LED, etc., that emits light of a certain colour when a respective one of the right ear and left ear is selected for testing and that emits light of a different colour when the other one of the right ear and left ear is selected for testing. For example, the light source may turn orange when the right ear is selected for testing and the light source may turn blue when the left ear is selected for testing. Alternatively or additionally, the light source may blink in a certain sequence to indicate selection of a respective left or right ear.

The light source may be integrated with the mechanically non-latching switch.

Instead, or additionally, the indicator may provide acoustic indication of the selected ear, e.g. the indicator may comprise a sound source, such as a miniature loudspeaker, that emits a specific sound when a respective one of the right ear and left ear is selected for testing and that emits a different sound when the other one of the right ear and left ear is selected for testing. The two sounds may be pure tones of different frequencies, or short different melodies, or different sequences of the same sound, e.g. one beep when the right ear is selected for testing and two beeps when the left ear is selected for testing.

The indicator may also be configured to indicate when a measurement is in progress, and/or air leakage and/or instability in the measured ear cavity volume, etc.

Instead, or additionally, indication of any of the above conditions indicated by the indicator of the remote user interface may be provided by the audiologic test apparatus, e.g. by respective indications displayed on a screen of the audiologic test apparatus, e.g. in well-known ways.

Instead, or additionally, indication of any of the above conditions indicated by the indicator of the remote user interface may be provided by the ear probe.

The remote user interface housing may be intended to rest at the patient's body, e.g. the remote user interface may be connected to the audiologic test apparatus with a cable that may rest together with the remote user interface housing across a shoulder of the patient.

The mechanically non-latching switch for selection of the right ear or the left ear of the patient for testing with the audiologic test apparatus may be duplicated, i.e. a housing of the audiologic test apparatus different from the remote user interface housing may also accommodate a user interface for selection of the right ear or the left ear of the patient for testing with the audiologic test apparatus so that an operator of the audiologic test apparatus has the option of selecting the left ear or the right ear for testing or for studying recorded test results relating to the selected ear either with the remote user interface or with the user interface of the audiologic test apparatus.

The indicator may also be duplicated, i.e. the housing of the audiologic test apparatus may also accommodate an indicator, e.g. of the types mentioned above with relation to the remote user interface, so that the ear currently selected may be indicated both at the housing of the audiologic test apparatus and at the remote user interface housing.

The audiologic test apparatus may be a self-contained unit with its own user interface with switches and a display, possibly a keyboard, as is well-known in the art of audiologic test apparatuses.

The audiologic test apparatus may also be based on a general purpose computer, such as a PC, laptop, PDA, smartphone, etc., for recording to the test results and that comprises the user interface of the audiologic test apparatus and that is interconnected with and controls a special purpose test apparatus with the required measurement circuitry and devices for performing the audiologic tests.

The recorded test results may be stored together with the identification of the right ear or the left ear that the stored test results relate to in the audiologic test apparatus and possibly other relevant information, such as the date, time, patient ID, operator ID, etc.

The recorded data may be transferred to another device, such as a remote storage, such as a server interconnected with the audiologic test apparatus through a Wide Area Network, such as the Internet.

The ear probe may be connected with a cable to the audiologic test apparatus in a well-known way.

The cable may include electrical conductors for interconnection of a microphone and a loudspeaker and switches and possible indicators, respectively, accommodated in the probe housing.

The cable may also have an air conduit for interconnection with a pump in the audiologic test apparatus for control of the air pressure in the ear canal subjected to clinical testing or screening in a way well-known in the art of audiologic testing.

Likewise, the ear probe may be connected to the remote user interface housing with a cable and the remote user interface housing may be connected to the audiologic test apparatus with a cable so that the ear probe may be connected with the audiologic test apparatus via the remote user interface housing.

The probe housing may constitute the remote user interface housing.

The ear probe may for example be used for acoustic impedance and static pressure measurements in the ear canal. These measurements, possibly in combination with audiometry, such as pure tone audiometry or speech audiometry, allow a variety of middle ear and other disorders to be diagnosed.

The ear probe preferably comprises a conventional ear probe tip that is preferably configured for fitting with an ear tip for insertion into an ear canal of the patient and for sealing the ear canal with an air-tight seal.

The ear probe tip may provide an output of an air conduit connected with the air conduit of the cable to the audiologic test apparatus.

The ear tip may be disposable and for example made of rubber.

Screening tests may include tympanometry, Ipsi and Contra acoustic reflex, Ipsi and Contra fast acoustic reflex threshold, pure tone audiometry, speech audiometry, etc.

Clinical tests may include tympanometry, Eustachian tube function, Ipsi and Contra manual acoustic reflex, Ipsi and Contra reflex decay, acoustic reflex latency, pure tone audiometry, speech audiometry, Gellé's test, etc.

Preferably, the audiologic test apparatus is configured for performing tests involving measurement and/or emission of sound.

The results of the ear measurements performed with the audiologic test apparatus and the ear probe are recorded by the audiologic test apparatus and stored in a memory of the audiologic test apparatus. The results are stored together with an indication of whether the right ear or the left ear was subject to the testing as selected with the remote user interface.

### BRIEF DESCRIPTION OF THE DRAWINGS

The drawings illustrate the design and utility of embodiments, in which similar elements are referred to by common reference numerals. These drawings may or may not be drawn to scale. In order to better appreciate how the above-recited and other advantages and objects are obtained, a more particular description of the embodiments will be rendered, which are illustrated in the accompanying drawings. These drawings depict only exemplary embodiments and are not therefore to be considered limiting in the scope of the claims.

In the drawings:
- Fig. 1: schematically shows a perspective view of a hand-held ear probe with a remote user interface for an audiologic apparatus utilized for testing an ear of a human, and
- Fig. 2: schematically shows a perspective view of a remote user interface housing for remote control of an audiologic apparatus.

### DETAILED DESCRIPTION OF THE DRAWINGS

Various illustrative examples of the new remote user interface according to the appended claims will now be described more fully hereinafter with reference to the accompanying drawings, in which various embodiments of new remote user interface are illustrated. The new remote user interface according to the appended claims may, however, be embodied in different forms and should not be construed as limited to the embodiments set forth herein. In addition, an illustrated embodiment needs not have all the aspects or advantages shown. An aspect or an advantage described in conjunction with a particular embodiment is not necessarily limited to that embodiment and can be practiced in any other examples even if not so illustrated, or if not so explicitly described. It should also be noted that the accompanying drawings are schematic and simplified for clarity, and they merely show details which are essential to the understanding of the new remote user interface, while other details have been left out.

As used herein, the singular forms "a," "an," and "the" refer to one or more than one, unless the context clearly dictates otherwise.

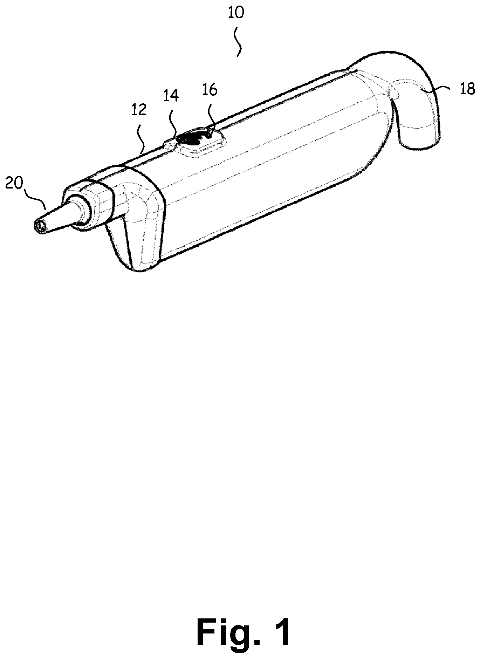

Fig. 1 shows schematically and in perspective a hand-held ear probe 10 with a probe housing 12 and configured for connection with an audiologic apparatus (not shown) and comprising a remote user interface 14 for operation of the audiologic apparatus. The remote user interface 14 comprises a mechanically non-latching switch 16 for selection of the right ear or the left ear of the patient for testing with the ear probe and the audiologic apparatus.

In this way, the operator of the ear probe need not move to the audiologic test apparatus during testing for selection of the right ear or the left ear for testing with the audiologic test apparatus. The mechanically non-latching switch may be a push button with appropriate tactile feedback, when the mechanically non-latching switch is actuated, i.e. depressed, in order to perform a selection of the right or left ear.

The illustrated mechanically non-latching switch 16 is configured for alternate selection of the right ear or the left ear for testing by repeated actuation of the mechanically non-latching switch. The mechanically non-latching switch 16 may be a push button, e.g. with appropriate tactile feedback, when the mechanically non-latching switch 16 is actuated, i.e. depressed, in order to perform a selection of the right or left ear.

In another embodiment (not shown), the mechanically non-latching switch 16 may be a touch sensitive switch, e.g. forming part of the surface of the probe housing 12, and preferably the surface of the touch sensitive switch is flush with the surrounding surface of the probe housing 12.

In another embodiment (not shown), the probe housing 12 accommodates two mechanically non-latching switches, one for selection of the right ear and one for selection of the left ear, respectively.

In another embodiment (not shown), the probe housing 12 also accommodates a mechanically non-latching switch configured for controlling start and stop of testing with the audiologic apparatus.

The illustrated mechanically non-latching switch 16 comprises a lamp (not visible) that illuminates the mechanically non-latching switch from inside the mechanically non-latching switch with light of different colours in response to the actual selection of the ear to be tested so that the illustrated mechanically non-latching switch 16 also constitutes an indicator of the one of the right ear or left ear that is currently selected for testing.

The lamp of the illustrated mechanically non-latching switch 16 emits orange light when the right ear is selected for testing and emits blue light when the left ear is selected for testing.

In another embodiment (not shown), the indicator may comprise a sound source, such as a miniature loudspeaker, instead of a light source, or in addition to the light source, wherein the sound source emits a specific sound when a respective one of the right ear and left ear is selected for testing and that emits a different sound when the other one of the right ear and left ear is selected for testing. The two different sounds may be pure tones of different frequencies, or short different melodies, or different sequences of the same sound, e.g. one beep when the right ear is selected for testing and two beeps when the left ear is selected for testing.

In another embodiment (not shown), the probe housing 12 accommodates a lamp configured to indicate measurement status, such as measurement is in progress, air leakage, instability of the measured ear cavity volume, etc.

During testing with the audiologic test apparatus (not shown), the ear probe 10 is connected with a cable 18 to the audiologic test apparatus in a well-known way. The cable 18 includes electrical conductors (not visible) for interconnection of a microphone (not visible) and a loudspeaker (not visible) and the mechanically non-latching switch and indicator 16 accommodated in the probe housing 12. The cable also 18 has an air conduit (not visible) for interconnection with a pump (not shown) in the audiologic test apparatus (not shown) for control of the air pressure in the ear canal subjected to clinical testing or screening in a way well-known in the art of audiologic testing.

Typically, the hand-held ear probe 10 is used for acoustic impedance and static pressure measurements in the ear canal. These measurements, possibly in combination with pure tone audiometry or speech audiometry, allow a variety of middle ear and other disorders to be diagnosed.

The ear probe 10 comprises a conventional ear probe tip 20 preferably configured for fitting with an ear tip (not shown) for insertion into an ear canal of the patient and for sealing the ear canal with an air-tight seal. The probe tip 20 is connected with the air conduit of the cable 18 and the pump (not shown) of the audiologic test apparatus (not shown).

Screening tests may include tympanometry, Ipsi and Contra acoustic reflex, Ipsi and Contra fast acoustic reflex threshold, pure tone audiometry, speech audiometry, etc.

Clinical tests may include tympanometry, Eustachian tube function, Ipsi and Contra manual acoustic reflex, Ipsi and Contra reflex decay, acoustic reflex latency, pure tone audiometry, speech audiometry, Gellé's test, etc.

The results of the ear measurements performed with audiologic test apparatus and the ear probe are recorded by the audiologic test apparatus and stored in a memory of the audiologic test apparatus. The results are stored together with an indication of whether the right ear or the left ear was subject to the testing as selected with the remote user interface.

When clinical or screening tests are performed, there is a need for recording which of the left ear or the right ear that is subjected to the test with the ear probe.

For this purpose, the mechanically non-latching switch 16 is conveniently provided at the hand-held ear probe 10 for selection of which of the left ear or the right ear that is to be subjected to the next audiologic test. For example, a reflex test may be performed, wherein the left ear is selected to be tested as ipsi and these selections may be displayed on a screen of the audiologic test apparatus as "Reflex: Left".

In this way, the operator of the ear probe 10 may perform selection of the ear to be subjected to the next test without having to move proximate to the audiologic test apparatus connected with the ear probe for the purpose of ear selection.

For example, when operating a hand held ear probe, the operator may have established an air-tight seal of the ear canal with the hand held ear probe before recognizing the need to select the ear in question before the test can be performed. Conventionally, the operator then had to remove the hand held ear probe from the ear to be tested in order to go to the audiologic test apparatus to perform the selection of the right or left ear and revert to the patient and re-establish the seal. This is avoided with the new hand-held ear probe 10 with its remote user interface 14.

Thus, cumbersome and time consuming operator movement is avoided between the audiologic test apparatus and the patient in order to select which ear that is going to be tested and in order to select the other ear when testing of the first ear has been performed. Time is saved and communication with the patient is maintained and disturbances are minimized during audiologic testing with the hand-held ear probe 10.

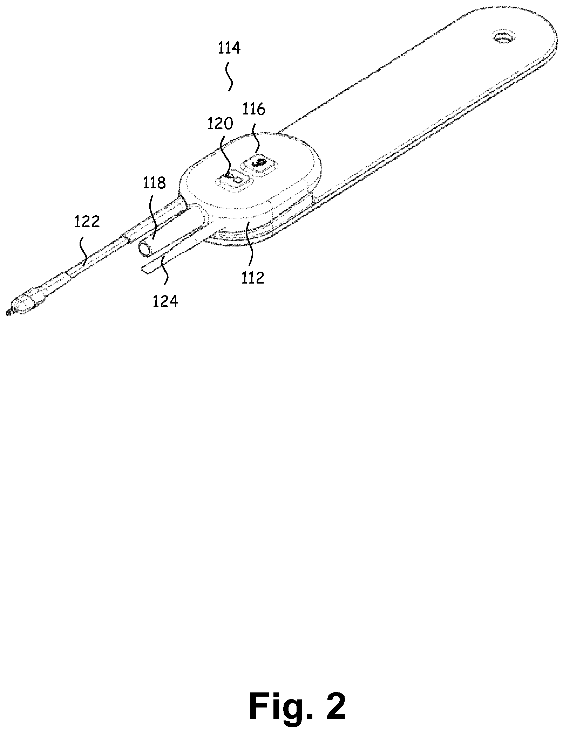

Fig. 2 shows schematically and in perspective a remote user interface housing 112 that accommodates the remote user interface 114 and is intended to rest at the patient's body. The remote user interface 114 is connected to the audiologic test apparatus (not shown) through port 118 with a cable (not shown) that may rest together with the remote user interface housing 112 across a shoulder (not shown) of the patient.

The ear probe (not shown) to be inserted in the selected ear may be connected with a cable 124 (partly shown) to the remote user interface housing 112 in a well-known way. The ear probe cable 124 includes electrical conductors for interconnection of a microphone and a loudspeaker and switches and possible indicators, respectively, accommodated in the probe housing. The ear probe cable 124 also has an air conduit for interconnection with a pump in the audiologic test apparatus for control of the air pressure in the ear canal subjected to clinical testing or screening in a way well-known in the art of audiologic testing.

When required, a second ear probe (not shown) to be inserted into the other ear than the selected ear may be connected with cable 122 to the remote user interface housing 112 in a well-known way. The cable 112 may be similar to the cable 124.

The remote user interface housing 112 assists in maintaining the various cables 122, 124 in convenient and appropriate locations during performance of the clinical or screening tests.

The remote user interface 114 operates with the same means and in the same way as the remote user interface 14 explained above in connection with Fig. 1.

In addition to the advantages of the remote user interface mentioned above with relation to Fig. 1, provision of the remote user interface 114 in a separate remote user interface housing 112 leads to the advantage that no force is applied to the ear probe when the remote user interface 114 accommodated in the remote user interface housing 112 is used, e.g. when the mechanically non-latching switch 116 for ear selection is depressed and released, thereby avoiding that the ear probe is inadvertently moved away from a desired position, e.g. breaking a previously established air tight seal.

The illustrated remote user interface 114 also comprises a mechanically non-latching switch 120 configured for controlling start and stop of testing with the audiologic apparatus in a way well-known in the art.

Although particular embodiments have been shown and described, it will be understood that it is not intended to limit the claimed inventions to the preferred embodiments, and it will be obvious to those skilled in the art that various changes and modifications may be made without department from the scope of the present invention as defined in the appended claims.

## Claims

1. A remote user interface (114) for connection with an audiologic test apparatus with an ear probe for audiologic testing of a patient, comprising
a remote user interface housing (112) accommodating a first mechanically non-latching switch (116) for alternating selection of one of the right ear and left ear of a patient for testing with the audiologic test apparatus,
wherein the remote user interface housing is different from a housing of the audiologic test apparatus; and
wherein the remote user interface housing is configured for mechanical connection with the ear probe of the audiologic test apparatus in such a way that the remote user interface will be located proximate the patient during the audiologic testing of the patient, and
**characterized in that**
the remote user interface housing accommodates a second mechanically non-latching switch (120) configured for controlling start and stop of testing with the audiologic test apparatus.

2. A remote user interface according to claim 1, wherein at least one of the first and second mechanically non-latching switches is a touch sensitive switch.

3. A remote user interface according to claim 2, wherein the touch sensitive switch is positioned so that a surface of the touch sensitive switch is flush with a surface of the remote user interface housing.

4. A remote user interface according to claim 1, wherein at least one of the first and second mechanically non-latching switches is a push button with tactile feedback.

5. A remote user interface according to any of the previous claims, wherein the remote user interface housing accommodates an indicator for indication of the one of the right ear and left ear that is currently selected for testing with the audiologic test apparatus.

6. A remote user interface according to claim 5, wherein the indicator provides visual indication of the respective ear selected for testing with the audiologic test apparatus.

7. A remote user interface according to claim 6, wherein the indicator comprises a light source that emits light of a certain colour when one of the right ear and left ear is selected for testing with the audiologic test apparatus and that emits light of a different colour when the other one of the right ear and left ear is selected for testing with the audiologic test apparatus.

8. A remote user interface according to any of claims 5-7, wherein the indicator comprises a sound source that emits a specific sound when one of the right ear and left ear is selected for testing with the audiologic test apparatus.

9. A remote user interface according to any of the previous claims, and connected to the audiologic test apparatus with a cable and to the ear probe with another cable so that the ear probe may be connected with the audiologic test apparatus via the remote user interface housing.

10. A remote user interface according to any of the previous claims, wherein the remote user interface housing constitutes at least a part of a housing of the ear probe.

11. A system comprising a remote user interface according to any of the previous claims and an audiologic test apparatus with an ear probe for audiologic testing of a patient for connection with the remote user interface, wherein the audiologic test apparatus has a housing different from the remote user interface housing, wherein the housing of the audiologic test apparatus accommodates a user interface for selection of the right ear or the left ear of the patient for testing with the audiologic test apparatus.

12. A system according to claim 11, wherein the housing of the audiologic test apparatus accommodates an indicator for indication of the selected ear.

13. A system according to claim 11 or 12, configured for storing test results of the audiologic test of the selected ear together with identification of the selected ear.

## Patentansprüche

1. Fernbenutzerschnittstelle (114) zur Verbindung mit einer audiologischen Testeinrichtung mit einer Ohrsonde für audiologische Testung eines Patienten, umfassend
ein Fernbenutzerschnittstellengehäuse (112), das einen ersten mechanisch nicht-verriegelnden Schalter (116) zur abwechselnden Auswahl eines des rechten Ohrs und des linken Ohrs eines Patienten für Testung mit der audiologischen Testeinrichtung beherbergt,
wobei sich das Fernbenutzerschnittstellengehäuse von einem Gehäuse der audiologischen Testeinrichtung unterscheidet; und
wobei das Fernbenutzerschnittstellengehäuse derart für eine mechanische Verbindung mit der Ohrsonde der audiologischen Testeinrichtung konfiguriert ist, dass sich die Fernbenutzerschnittstelle während der audiologischen Testung des Patienten in der Nähe des Patienten befindet, und
**dadurch gekennzeichnet, dass**
das Fernbenutzerschnittstellengehäuse einen zweiten mechanisch nicht-verriegelnden Schalter (120) beherbergt, der dazu konfiguriert ist, Start und Stopp der Testung mit der audiologischen Testeinrichtung zu steuern.

2. Fernbenutzerschnittstelle nach Anspruch 1, wobei mindestens einer des ersten und des zweiten mechanisch nicht-verriegelnden Schalters ein berührungsempfindlicher Schalter ist.

3. Fernbenutzerschnittstelle nach Anspruch 2, wobei der berührungsempfindliche Schalter so positioniert ist, dass eine Oberfläche des berührungsempfindlichen Schalters bündig mit einer Oberfläche des Fernbenutzerschnittstellengehäuses ist.

4. Fernbenutzerschnittstelle nach Anspruch 1, wobei mindestens einer des ersten und des zweiten mechanisch nicht-verriegelnden Schalters ein Druckknopf mit taktiler Rückmeldung ist.

5. Fernbenutzerschnittstelle nach einem der vorstehenden Ansprüche, wobei das Fernbenutzerschnittstellengehäuse einen Indikator zur Anzeige des einen des rechten Ohrs und des linken Ohrs beherbergt, das aktuell zur Testung mit der audiologischen Testeinrichtung ausgewählt ist.

6. Fernbenutzerschnittstelle nach Anspruch 5, wobei der Indikator eine visuelle Anzeige des jeweiligen Ohrs bereitstellt, das zur Testung mit der audiologischen Testeinrichtung ausgewählt ist.

7. Fernbenutzerschnittstelle nach Anspruch 6, wobei der Indikator eine Lichtquelle umfasst, die Licht einer bestimmten Farbe aussendet, wenn eines des rechten Ohrs und des linken Ohrs zur Testung mit der audiologischen Testeinrichtung ausgewählt wird, und die Licht einer anderen Farbe aussendet, wenn das andere des rechten Ohrs und des linken Ohrs zur Testung mit der audiologischen Testeinrichtung ausgewählt wird.

8. Fernbenutzerschnittstelle nach einem der Ansprüche 5-7, wobei der Indikator eine Tonquelle umfasst, die einen bestimmten Ton aussendet, wenn eines des rechten Ohrs und des linken Ohrs zur Testung mit der audiologischen Testeinrichtung ausgewählt wird.

9. Fernbenutzerschnittstelle nach einem der vorstehenden Ansprüche und über ein Kabel mit der audiologischen Testeinrichtung und über ein anderes Kabel mit der Ohrsonde verbunden, so dass die Ohrsonde mittels des Fernbenutzerschnittstellengehäuses mit der audiologischen Testeinrichtung verbunden werden kann.

10. Fernbenutzerschnittstelle nach einem der vorstehenden Ansprüche, wobei das Fernbenutzerschnittstellengehäuse mindestens einen Teil eines Gehäuses der Ohrsonde bildet.

11. System, das eine Fernbenutzerschnittstelle nach einem der vorstehenden Ansprüche und eine audiologische Testeinrichtung mit einer Ohrsonde zur audiologischen Testung eines Patienten zur Verbindung mit der Fernbenutzerschnittstelle umfasst, wobei die audiologische Testeinrichtung ein Gehäuse aufweist, das sich von dem Fernbenutzerschnittstellengehäuse unterscheidet, wobei das Gehäuse der audiologischen Testeinrichtung eine Benutzerschnittstelle zur Auswahl des rechten Ohrs oder des linken Ohrs des Patienten zur Testung mit der audiologischen Testeinrichtung beherbergt.

12. System nach Anspruch 11, wobei das Gehäuse der audiologischen Testeinrichtung einen Indikator zur Anzeige des ausgewählten Ohrs beherbergt.

13. System nach Anspruch 11 oder 12, das zum Speichern von Testergebnissen der audiologischen Testung des ausgewählten Ohrs zusammen mit einer Kennung des ausgewählten Ohrs konfiguriert ist.

## Revendications

1. Interface utilisateur à distance (114) destinée à être raccordée à un appareil de test audiologique avec une sonde auriculaire pour un test audiologique d'un patient, comprenant
un boîtier (112) d'interface utilisateur à distance contenant un premier commutateur sans verrouillage mécanique (116) pour la sélection alternée de l'une de l'oreille droite et de l'oreille gauche d'un patient à tester avec l'appareil de test audiologique,
dans laquelle le boîtier d'interface utilisateur à distance est différent d'un boîtier de l'appareil de test audiologique ; et
dans laquelle le boîtier d'interface utilisateur à distance est configuré pour un raccordement mécanique avec la sonde auriculaire de l'appareil de test audiologique de telle manière que l'interface utilisateur à distance sera située à proximité du patient pendant le test audiologique du patient, et
**caractérisée en ce que**
le boîtier d'interface utilisateur à distance contient un second commutateur sans verrouillage mécanique (120) configuré pour commander le démarrage et l'arrêt du test avec l'appareil de test audiologique.

2. Interface utilisateur à distance selon la revendication 1, dans laquelle au moins l'un des premier et second commutateurs sans verrouillage mécanique est un commutateur tactile.

3. Interface utilisateur à distance selon la revendication 2, dans laquelle le commutateur tactile est positionné de telle sorte qu'une surface du commutateur tactile affleure une surface du boîtier d'interface utilisateur à distance.

4. Interface utilisateur à distance selon la revendication 1, dans laquelle au moins l'un des premier et second commutateurs sans verrouillage mécanique est un bouton poussoir à retour tactile.

5. Interface utilisateur à distance selon l'une quelconque des revendications précédentes, dans laquelle le boîtier d'interface utilisateur à distance contient un indicateur pour indiquer celle de l'oreille droite et de l'oreille gauche qui est actuellement sélectionnée pour le test avec l'appareil de test audiologique.

6. Interface utilisateur à distance selon la revendication 5, dans laquelle l'indicateur fournit une indication visuelle de l'oreille respective sélectionnée pour le test avec l'appareil de test audiologique.

7. Interface utilisateur à distance selon la revendication 6, dans laquelle l'indicateur comprend une source de lumière qui émet de la lumière d'une certaine couleur lorsque l'une de l'oreille droite et de l'oreille gauche est sélectionnée pour le test avec l'appareil de test audiologique et qui émet de la lumière d'une couleur différente lorsque l'autre de l'oreille droite et de l'oreille gauche est sélectionnée pour le test avec l'appareil de test audiologique.

8. Interface utilisateur à distance selon l'une quelconque des revendications 5-7, dans laquelle l'indicateur comprend une source sonore qui émet un son spécifique lorsque l'une de l'oreille droite et de l'oreille gauche est sélectionnée pour le test avec l'appareil de test audiologique.

9. Interface utilisateur à distance selon l'une quelconque des revendications précédentes, et raccordée à l'appareil de test audiologique avec un câble et à la sonde auriculaire avec un autre câble de sorte que la sonde auriculaire puisse être raccordée à l'appareil de test audiologique par l'intermédiaire du boîtier d'interface utilisateur à distance.

10. Interface utilisateur à distance selon l'une quelconque des revendications précédentes, dans laquelle le boîtier d'interface utilisateur à distance constitue au moins une partie d'un boîtier de la sonde auriculaire.

11. Système comprenant une interface utilisateur à distance selon l'une quelconque des revendications précédentes et un appareil de test audiologique avec une sonde auriculaire pour le test audiologique d'un patient, destiné à être raccordé à l'interface utilisateur à distance, dans lequel l'appareil de test audiologique présente un boîtier différent du boîtier d'interface utilisateur à distance, dans lequel le boîtier de l'appareil de test audiologique contient une interface utilisateur pour la sélection de l'oreille droite ou de l'oreille gauche du patient à tester avec l'appareil de test audiologique.

12. Système selon la revendication 11, dans lequel le boîtier de l'appareil de test audiologique contient un indicateur pour indiquer l'oreille sélectionnée.

13. Système selon la revendication 11 ou 12, configuré pour stocker des résultats de test du test audiologique de l'oreille sélectionnée ainsi que l'identification de l'oreille sélectionnée.
